# EUROPÄISCHE PATENTSCHRIFT

(11) **EP 2 124 783 B1**
(45) Veröffentlichungstag und Bekanntmachung des Hinweises auf die Patenterteilung: **11.04.2018**
(21) Anmeldenummer: 08717088.2
(22) Anmeldetag: 25.02.2008
(51) Int. Cl.: A61B 17/74

(54) **IMPLANTAT ZUR FRAKTURVERSORGUNG**
IMPLANT FOR FRACTURE CARE
IMPLANT POUR LE TRAITEMENT D'UNE FRACTURE

(30) Priorität: 23.02.2007 CH 309072007
(43) Veröffentlichungstag der Anmeldung: 02.12.2009
(73) Patentinhaber: Zimmer GmbH, 8404 Winterthur (CH)
(72) Erfinder: KELLER, Samuel, CH-8442 Hettlingen (CH); GARIC, Miodrag, CH-8408 Winterthur (CH); STRICKER, Hermann, CH-8911 Rifferswil (CH); SENGER, Reto, CH-8409 Winterthur (CH); BÜHLER, Daniel, CH-8542 Wiesendangen (CH); SCHWAMMBERGER, Andreas Eduard, CH-4433 Ramlinsburg (CH); LONGSWORTH, Serene, Randolph, New Jersey 07869 (US)
(74) Vertreter: Manitz Finsterwald Patentanwälte PartmbB
(86) Internationale Anmeldenummer: PCT/EP2008/052254
(87) Internationale Veröffentlichungsnummer: WO 2008/102016

(56) Entgegenhaltungen:
- WO-A1-2007/016796
- WO-A1-2008/014742
- US-A- 2 381 050
- US-A- 5 282 861
- US-A1- 2003 045 885
- US-A1- 2003 083 662
- US-A1- 2007 173 838

## Beschreibung

Die Erfindung betrifft ein Implantat zur Frakturversorgung der im Anspruch 1 beschriebenen Art. Sie betrifft weiterhin ein Implantatsystem zur Frakturversorgung.

Zur Versorgung einer proximalen Femurfraktur (sub- oder intertrochantäre Fraktur oder Schenkelhalsfraktur) mit einem Implantat wird üblicherweise ein intramedullärer Marknagel in den Femurknochen eingebracht und der Femurkopf über eine im proximalen Abschnitt des Marknagels gelagerte Schenkelhalsschraube fixiert. Die Schenkelhalsschraube ist hierzu durch eine Schrägbohrung im proximalen Abschnitt des Marknagels geführt und in der Spongiosa des Femurkopfes durch Einschrauben verankert. Proximal bedeutet hierbei näher am Körper bzw. Herzen eines Patienten, während distal das Gegenteil bedeutet.

Schenkelhalsfrakturen werden weiterhin häufig versorgt, indem eine Platte mit einer in Richtung des Schenkelhalses weisenden Hülse lateral am Femur fixiert wird, wobei die Hülse medial im Femur zu liegen kommt. In der Hülse wird eine Schenkelhalsschraube gleitend geführt, die mit ihrem proximalen Ende im Femurkopf verankert ist.

Um eine stabile Verankerung der Schenkelhalsschraube zu erzielen, wird insbesondere versucht, durch eine besondere Ausgestaltung des Gewindes der Schenkelhalsschraube eine stabile und auch verdrehsichere Verankerung der Schraube im Femurkopf zu erreichen. Es wurde auch schon vorgeschlagen, sowohl eine Schraube als auch einen Stift zur Verdrehsicherung parallel in den Femurkopf einzubringen. Die Verankerung einer Schenkelhalsschraube vor allem in osteoporotischen Knochen ist nicht ohne Schwierigkeiten zu bewerkstelligen.

Auch bei anderen Indikationen zur Frakturversorgung muss eine Osteosyntheseschraube in Knochengewebe mit geringer Stabilität verankert werden; zu nennen wäre beispielhaft der proximale Humerus. Weiterhin soll bei Implantaten zur Frakturversorgung auch möglichst eine Explantation des Implantats nach erfolgter Heilung möglich sein, was im Vergleich zu prothetischen Anwendungen eine zusätzliche Randbedingung aufbringt.

Aus der WO96/39974 ist eine Prothese zum Auffüllen eines Kanals im Femurkopfkern bekannt. Diese Prothese weist ein poröses Füllteil zum Auffüllen des Kanals auf. Dieses ist vorgesehen, um sich mit dem Knochengewebe zu verbinden. Damit wird nekrotische Knochensubstanz verstärkt.

In der US2003/0045885 A1 ist ein rohrförmiges Implantat beschrieben, das beispielsweise selbstschneidend im Knochen verankert werden kann. Die Oberfläche dieses Implantats kann porös ausgebildet sein, um sich besser in das Knochengewebe zu integrieren. Das Implantat dient der Verstärkung von osteoporotischem Knochengewebe im Bereich des Schenkelhalses.

Aus der US 3,852,045 geht ein intramedulläres Implantat für eine Hüftgelenksprothese hervor, welche einen festen und strukturell belastbaren zentralen Bereich aufweist, sowie fest verbundene poröse Bereiche an jedem axialen Ende.

Nachfolgend wird ein verbessertes Implantat zur Frakturversorgung der eingangs genannten Art angegeben, welches neben weiteren vorteilhaften Wirkungen eine verbesserte Verankerung im Knochengewebe ermöglicht.

Dies, neben einer Vielzahl weiterer vorteilhafter Wirkungen, vermag ein Implantat zur Frakturversorgung mit den Merkmalen von Anspruch 1 zu leisten.

Dieses Implantat zeichnet sich dadurch aus, dass es einen länglichen und insbesondere im Wesentlichen rotationssymmetrischen Grundkörper und einen Verankerungsabschnitt umfasst, wobei der Verankerungsabschnitt wenigstens teilweise eine Außenfläche aus einem porösen Werkstoff aufweist, und wobei der Verankerungsabschnitt in einem axialen Endbereich des Implantats angeordnet ist und lösbar mit dem Grundkörper verbunden ist beziehungsweise verbindbar ist. Unter "verbindbar" versteht der Fachmann dabei unzweideutig, dass der Grundkörper und der Verankerungsabschnitt beispielsweise optional, zum Beispiel vor der Implantation, als getrennte Elemente vorliegen, aber bestimmungsgemäss mittels einer lösbaren Verbindung zum Implantat zusammengesetzt werden können, und auch entsprechend ausgestaltet sind. Eine lösbare Verbindung bedeutet in diesem Zusammenhang insbesondere, dass die Verbindung ohne strukturelle Schäden an einer der verbundenen Komponenten getrennt werden kann; beispielsweise wäre hier eine Schraubenverbindung zu nennen. Der Grundkörper ist beispielsweise ein Implantatschaft. Der Grundkörper ist beispielsweise aus Titan, Edelstahl, einem Kompositwerkstoff und dergleichen hergestellt, und gewährleistet die strukturelle Stabilität der Implantats, derart, dass die Knochenfragmente während der Frakturheilung gegeneinander fixiert sind, während die Frakturstelle noch vor der vollständigen Knochenheilung eine gewisse Belastbarkeit aufweist. Der Verankerungsabschnitt weist beispielsweise eine Länge auf, die weniger als 30% oder 25% oder weniger als 20% oder sogar weiniger als 15% der gesamten Längserstreckung des Implantats entspricht. Es wird sozusagen eine Funktionentrennung erreicht: der Verankerungsabschnitt dient der guten Verankerung im Knochen, und gewährleistet eine grossflächige Verteilung auftretender Kräfte, sowie eine verbesserte Verankerung des Grundelements im Knochen, insbesondere bei einer schlechten Knochenqualität, wie sie insbesondere häufig im Bereich des Schenkelhalses, aber auch beispielsweise im proximalen Humerus angetroffen wird. Der Grundkörper hingegen ist aus einem kompakten Werkstoff aufgebaut und gewährleistet strukturelle Stabilität während der Frakturheilung. Dadurch, dass der Verankerungsabschnitt und der Grundkörper lösbar verbunden sind, kann das Implantat nach erfolgter Frakturheilung wieder explantiert werden, wobei nur der Verankerungsabschnitt fest mit dem Knochengewebe verbunden und im Körper verbleibt.

Gemäß einer Ausführungsform des vorgeschlagenen Gegenstandes kann das Implantat als Schenkelhalsimplantat oder -schraube ausgebildet sein, bei dem der Verankerungsbereich am proximalen Ende des Implantats angeordnet ist. Unter dem proximalen Ende des Implantats wird hierbei der Abschnitt verstanden, der im Femurkopf implantiert wird, d.h. der im Gegensatz zu einem distalen Abschnitt näher am Körper eines Patienten befindliche Abschnitt. Generell gilt hierin, dass proximal näher am Körper Patienten und distal zur Gliedmasse hin orientiert bedeutet. Im Falle einer Schenkelhalsschraube bedeutet das auch, dass das proximale Ende die Spitze der Schraube darstellt, während der Schraubenkopf am distalen Ende liegt. Wie bereits oben erläutert führt der Einsatz eines porösen und insbesondere biokompatiblen Werkstoffs im proximalen Bereich von Schenkelhalsimplantaten zu dem Vorteil, dass eine besonders stabile Verankerung des Implantats in der Spongiosa des Femurkopfs erzielt werden kann, was besonders für osteoporotische Knochen von großem Vorteil ist. Unter einer Außenfläche wird hier im Sinne der vorliegenden Offenbarung zumindest ein Teilbereich der gesamten Außenfläche des proximalen Endes des Schenkelhalsimplantats verstanden.

Festzuhalten ist, dass alle im Folgenden beschriebenen Ausführungsformen eines Implantats auch und insbesondere als Schenkelhalsimplantate, insbesondere zur Versorgung einer proximalen Femurfraktur oder einer Schenkelhalsfraktur, Verwendung finden können, wobei dann spezifisch der Verankerungsabschnitt an einem proximalen Ende des Implantats angeordnet ist, während der Grundkörper distal liegt. Allfällige Elemente zur Verbindung mit dem Verankerungsabschnitt sind dann am proximalen Ende des Grundkörpers, angeordnet.

Weiterhin kann der Verankerungsabschnitt jeweils als Hülse ausgebildet sein, derart, dass sich die den Verankerungsabschnitt betreffenden Merkmale der nachstehend beschriebenen Ausführungsformen auch jeweils auf eine derartige Hülse erstrecken.

Der poröse biokompatible Werkstoff kann gemäß einer Ausführungsform des vorgeschlagenen Implantats ein poröses Kohlenstoffsubstrat sein, das mit einem biokompatiblen Material beschichtet ist. Insbesondere kann gemäß einer Ausführungsform des vorgeschlagenen Implantats das biokompatible Material Tantal, Niobium oder eine Legierung mit Tantal und Niobium, insbesondere eine Legierung aus Niobium, Hafnium und/oder Wolfram mit Tantal oder Hafnium und/oder Wolfram mit Niobium, sein, und das biokompatible Material kann mittels einer Gasphasenabscheidung auf das hochgradig poröse Kohlenstoffsubstrat aufgebracht worden sein. Dadurch wird eine besonders große Kontaktfläche des Implantats mit Knochengewebe geschaffen, wodurch das Einwachsen des Implantats in die Spongiosa des Femurkopfs beschleunigt und die Integration des Implantats in die Spongiosa verbessert werden kann. Ein geeigneter Werkstoff ist ausführlich in der US 5,282,861 beschrieben. Ein derartiges Implantat besitzt gegenüber herkömmlichen Implantaten wie beispielsweise Schenkelhalsschrauben aus Titan den Vorteil, dass das Implantat zu einer besseren und vor allem stabileren Verankerung Knochen, beispielsweise im Femurkopf bzw. in der Spongiosa des Femurkopfes führt, da Knochengewebe in die Poren des porösen Werkstoffs einwachsen kann. Insbesondere wenn der poröse biokompatible Werkstoff zusätzlich das Knochenwachstum stimulieren kann, wird eine besonders stabile Verbindung von Knochengewebe und Implantat im Vergleich zu herkömmlichen Implantaten erreicht. Vor allem bei osteoporotischen Knochen, bei welchen die Verankerung eines Implantats aufgrund des brüchigen Knochengewebes in der Regel kritisch ist, kann das vorgeschlagene Implantat trotzdem zu einer stabilen Verankerung in einem Knochen wie beispielsweise dem Femurkopf aufgrund der wesentlich besseren Integration des Implantats in den Knochen führen. Unter einem porösen biokompatiblen Werkstoff wird insbesondere ein Werkstoff verstanden, wie er in der US 5,282,861 beschrieben ist.

Ein geeigneter poröser Werkstoff, der für die hier beschriebene Anwendung besonders geeignet ist, befindet sich unter dem Namen Trabecular Metal™ im Portfolio der Anmelderin.

In einer Ausführungsform des vorgeschlagenen Gegenstandes besteht der Verankerungsabschnitt vollständig aus dem porösen Werkstoff. Dabei wird der Grundkörper unmittelbar mit dem Verankerungsabschnitt lösbar verbunden, beispielsweise durch Einschrauben eines Gewindeabschnitts des Grundkörpers in eine Hülse aus dem porösen Werkstoff.

In einer anderen Ausführungsform umfasst der Verankerungsabschnitt ein Substrat, an dessen Aussenseite der poröse Werkstoff angeordnet und insbesondere unlösbar mit dem Substrat verbunden ist. Unter einer unlösbaren Verbindung ist dabei eine Verbindung zu verstehen, die im Wesentlichen ohne strukturelle Beschädigungen nicht trennbar ist. Hierunter fallen zum Beispiel stoffschlüssige Verbindungen oder auch Klebeverbindungen oder sogenannte Bondings. Hierunter sind weiterhin Ausführungsformen zu verstehen, bei denen der poröse Werkstoff direkt auf dem Substrat hergestellt wurde, derart, dass die Komponente sozusagen einstückig vorliegt. Im weiteren Sinne sind auch feste Pressverbindungen hierunter zu verstehen, wobei diese Aufzählung nicht abschliessend sein soll. Beispielsweise weist das Substrat Mittel zur lösbaren Verbindung mit dem Grundkörper auf, wobei die Mittel in einer Ausführungsform ein Innengewinde und/oder ein Aussengewinde an einem axialen Ende des Verankerungsabschnitts sind.

Eine Ausführungsform des hier vorgeschlagenen Gegenstandes zeichnet sich dadurch aus, dass der Grundkörper als Schaft ausgebildet ist, der an einem Ende mit einem Gewinde zum Verbinden mit dem Verankerungsabschnitt ausgebildet ist. Dabei kann es sich um ein Aussengewinde zum Einschrauben in den Verankerungsabschnitt oder auch um ein Innengewinde handeln. Insbesondere im Falle eines Aussengewindes erstreckt sich zwischen dem Schaft und dem Gewinde ein konischer, sich vom Gewinde zum Schaft hin im Durchmesser vergrösserender, Bereich. Der Bereich ist in einer Ausführungsform dafür vorgesehen, beim Einschrauben in einen Verankerungsabschnitt, insbesondere einen als Hülse ausgestalteten Verankerungsabschnitt, diesen aufzuspreizen.

In einer weiteren Ausführungsform des beschreibenen Gegenstandes weist der Verankerungsabschnitt an einem Aussenumfang ein Gewinde, insbesondere ein Spongiosagewinde, auf. Dabei kann das Implantat beispielsweise in Form einer hybriden Schenkelhalsschraube ausgebildet sein, die einen distal angeordneten Schaftbereich und einen mit dem porösen biokompatiblen Werkstoff versehenden proximalen Abschnitt aufweist, der ein Spongiosagewinde umfasst. Ein distaler Bereich des Implantats ist ferner insbesondere zur Lagerung und/oder Führung des Implantats in einem intramedullären Marknagel und/oder einer Führungshülse einer Hüftplatte ausgebildet. Eine derartige Schraube besitzt den Vorteil, dass sie in einem Arbeitsgang in den Femurkopf eingedreht werden kann, d.h. nach einem Vorbohren in den Femurkopf kann die hybride Schenkelhalsschraube direkt durch eine entsprechende Aufnahmebohrung eines intramedullären Marknagels geführt und in die vorbereitete Bohrung im Femurkopf eingedreht werden.

Selbstverständlich kann diese Form einer hybriden Osteosyntheseschraube analog auch zur Behandlung anderer Frakturen verwendet werden.

Weiterhin kann der Verankerungsabschnitt als Hülse ausgeführt sein. Dadurch ist der Verankerungsabschnitt leicht in den Knochen einbringbar , beispielsweise durch Einschlagen mit einem Hammer in eine Bohrung im Knochen.

Der Verankerungsabschnitt weist einer weiteren Ausführungsform in wenigstens einem axialen Bereich Längsschlitze oder Längsrillen auf. Dabei kann ein vorstehend beschriebener konischer Bereich des Grundkörpers dazu ausgebildet sein, um beim Einschrauben in die Hülse diese aufzuspreizen. Insbesondere ist dabei die axiale Erstreckung des Gewindes des Grundkörpers insbesondere kleiner ist als die Länge des Verankerungsabschnitts. Durch das Aufspreizen wird der Verankerungsabschnitt nach der Art eines Dübels in die Spongiosa hineingedrückt, was zu einer verbesserten Verankerung im Knochen resultiert.

Der Verankerungsabschnitt weist gemäß einer weiteren Ausführungsform einen geschlossenen und/oder einen abgerundeten Endabschnitt auf. Das geschlossene und/oder abgerundete Ende ist dabei an der dem Grundkörper abgewandten Seite des Verankerungsabschnitts angeordnet. Dies erleichtert zum Beispiel das Einbringen des Verankerungsabschnitts bzw. des Implantats in eine Bohrung.

Der Verankerungsabschnitt ist gemäß einer Ausführungsform im Ausgangszustand, d.h. im unbenutzten Zustand, an einer Innenseite glatt ausgeführt, derart, dass sich ein Aussengewinde des Grundkörpers beim erstmaligen Einschrauben, insbesondere bei der Implantation, ins Innere des Verankerungsabschnitts einschneidet. Dadurch ist der Verankerungsabschnitt beispielsweise mit unterschiedlichen Schraubengewinden verwendbar, und es resultiert eine sehr stabile Gewindeverbindung.

Eine weitere Ausführungsform des Implantats zeichnet sich dadurch aus, dass der Grundkörper des Implantats zur Lagerung und/oder Führung des Implantats in einer Querbohrung eines intramedullären Marknagels und/oder einer Führungshülse einer Frakturplatte ausgebildet ist.

Beschrieben sind weiterhin eine Schraube, insbesondere eine Schenkelhalsschraube, mit den den Grundkörper betreffenden Merkmalen der vorstehend beschriebenen Ausführungsformen, sowie ein Verankerungsabschnitt zur Verwendung in einem Implantat der vorstehend beschriebenen Art.

Ferner beschrieben ist eine Hülse zur Verwendung in einem Implantat der vorgeschlagenen Art, gekennzeichnet durch die eine Hülse und/oder einen Verankerungsabschnitt betreffenden Merkmale einer der vorstehenden Ausführungsformen des Implantats.

Beschrieben ist weiterhin ein Implantatsystem zur Frakturversorgung, umfassend einen Marknagel mit einer Durchführungsöffnung für ein Fixierungselement und ein als Fixierungslement verwendetes Implantat der vorstehend beschriebenen Art, wobei insbesondere der Marknagel ein proximaler Femurnagel und das Fixierungslement ein Schenkelhalsimplantat ist. Weiterhin betrifft demnach eine Ausführungsform ein Implantatsystem zur Frakturversorgung, umfassend einen Marknagel mit einer proximalen Durchführungsöffnung für ein Schenkelhalsimplantat und sowie ein Schenkelhalsimplantat nach einer der vorstehenden Ausführungsformen eines Implantats.

Ebenfalls beschrieben ist ein Implantatsystem zur Frakturversorgung, umfassend eine Platte zur Fixierung an einem Knochen und einer daran angeordneten Führungshülse zur gleitenden Aufnahme eines Fixierungsmittels und ein als Fixierungsmittel verwendetes Implantat der vorstehend beschriebenen Art. Gemäß einer spezifischeren Ausführungsform wird ferner ein Implantatsystem zur Frakturversorgung angegeben, welches eine Platte zur lateralen Fixierung am Femur und einer daran angeordneten Führungshülse zur gleitenden Aufnahme eines Schenkelhalsimplantats sowie ein Schenkelhalsimplantat nach einer der vorstehend beschriebenen Ausführungsformen eines Implantats umfasst, wobei insbesondere das Schenkelhalsimplantat in einem distalen Bereich ein axiales Innengewinde aufweist. Dieses ist als "Dynamische Hüftplatte" bekannt.

Weiterhin wird ein nicht-erfindungsgemässes Verfahren zur operativen Versorgung einer proximalen Femurfraktur oder einer Schenkelhalsfraktur angegeben, mit einem als Schenkelhalsimplantat verwendeten Implantat wie vorstehend beschrieben, bei dem
- ein intramedullärer Marknagel in den Femur eines Patienten oder eine Hüftplatte mit Führungshülsen in einen Patienten eingebracht wird,
- eine Bohrung zur zumindest teilweisen Aufnahme des Schenkelhalsimplantats in der Spongiosa des Femurkopfs eingebracht wird,
- das Schenkelhalsimplantat durch eine Schrägbohrung im proximalen Abschnitt des Marknagels oder durch eine Führungshülse der Hüftplatte geführt und ein proximaler, mit dem porösen biokompatiblen Werkstoff versehener Abschnitt des Schenkelhalsimplantats in die Bohrung im Femurkopf eingeschraubt wird oder eine mit dem porösen biokompatiblen Werkstoff versehene Hülse des Schenkelhalsimplantats in die Bohrung im Femurkopf eingeschlagen und eine Schenkelhalsschraube des Schenkelhalsimplantats durch eine Schrägbohrung im proximalen Abschnitt des Marknagels oder eine Führungshülse der Hüftplatte geführt und in die eingeschlagene Hülse eingeschraubt wird. Dabei wird insbesondere eine proximale Femurfraktur unter Zuhilfenahme eines Nagels und eine Schenkelhalsfraktur unter Zuhilfenahme der Ausführungsform mit einer Platte versorgt.

Es versteht sich von selbst, dass das oben angegebene nicht-erfindungsgemässe Verfahren auch analog auf die Behandlung anderer Frakturen mit einem Implantat beziehungsweise Implantatsystem wie oben angegeben übertragen werden kann. Selbstverständlich können die Merkmale der oben angegebenen Ausführungsformen des vorgeschlagenen Implantats jeweils untereinander zu spezifischen Ausführungsformen kombiniert werden.

Die Erfindung wird nachfolgend anhand von in den Zeichnungen dargestellten Ausführungsbeispielen näher erläutert. Im Einzelnen zeigen
- Fig. 1: ein erstes Ausführungsbeispiel eines Implantats, beispielsweise eines Schenkelhalsimplantats, in Form einer Kombination einer Schraube, beispielsweise einer Schenkelhalsschraube, und eines im Knochen verankerbaren Dübels;
- Fig. 2: in einer stark vergrößerten Ansicht einen Ausschnitt des hochgradig porösen Werstoffs, beispielsweise Tantalums, das gemäß dieser Offenbarung Anwendung findet;
- Fig. 3A-3D: verschiedene Ausführungsformen des Dübels des ersten Ausführungsbeispiels eines Implantats, insbesondere eines Schenkelhalsimplantats;
- Fig. 4: eine mit einem Schenkelhalsimplantat gemäß dem ersten Ausführungsbeispiel versorgte proximale Femurfraktur;
- Fig. 5: ein zweites Ausführungsbeispiel eines hier vorgeschlagenen Schenkelhalsimplantats in Form einer hybriden Schenkelhalsschraube;
- Fig. 6: eine mit einem Schenkelhalsimplantat gemäß dem zweiten Ausführungsbeispiel versorgte proximale Femurfraktur;
- Fig. 7: ein Schenkelhalsimplantat in Form einer Kombination einer Schenkelhalsschraube und einer im Femurkopf einschlagbaren und verankerbaren Hülse, in welche eine Schenkelhalsschraube eingeschraubt werden kann;
- Fig. 8: das Einbringen eines Schenkelhalsimplantats in den Femurkopf zur Versorgung einer proximalen Femurfraktur;
- Fig. 9: ein Implantat zur Versorgung einer Schenkelhalsfraktur umfassend eine Platte zur lateralen Fixierung am Femur und einer Führungshülse zur gleitenden Aufnahme eines Schenkelhalsimplantats;
- Fig. 10: einen Querschnitt durch den Verankerungsabschnitt eines Schenkelhalsimplantats gemäß einem weiteren Ausführungsbeispiel;
- Fig. 11: einen Querschnitt durch das Schenkelhalsimplantat aus der Figur 10 im zusammengesetzten Zustand ; und
- Fig. 12: eine perspektivische Darstellung des mehrteiligen Schenkelhalsimplantats gemäß der Figuren 10 und 11.

Die nachfolgende Beschreibung der Ausführungsbeispiele und die Zeichnungen dienen dem besseren Verständnis der Erfindung und sollen nicht zur Einschränkung des in den Ansprüchen gekennzeichneten Gegenstands herangezogen werden. Dabei wird die Erfindung im Wesentlichen am Beispiel von Schenkelhalsimplantaten erläutert, wobei dem Fachmann die Übertragung auf Implantate der beschriebenen und beanspruchten Art zur Versorgung anderer Frakturen, wir zum Beispiel Frakturen des proximalen Humerus, ohne Weiters möglich ist und die dementsprechende Offenbarung implizit in den Ausführungsbeispielen mit enthalten ist. Insbesondere wird dabei der Aufbau des beanspruchten Implantats hinsichtlich der wesentlichen in den Ansprüchen enthalteten Merkmale nicht verändert.

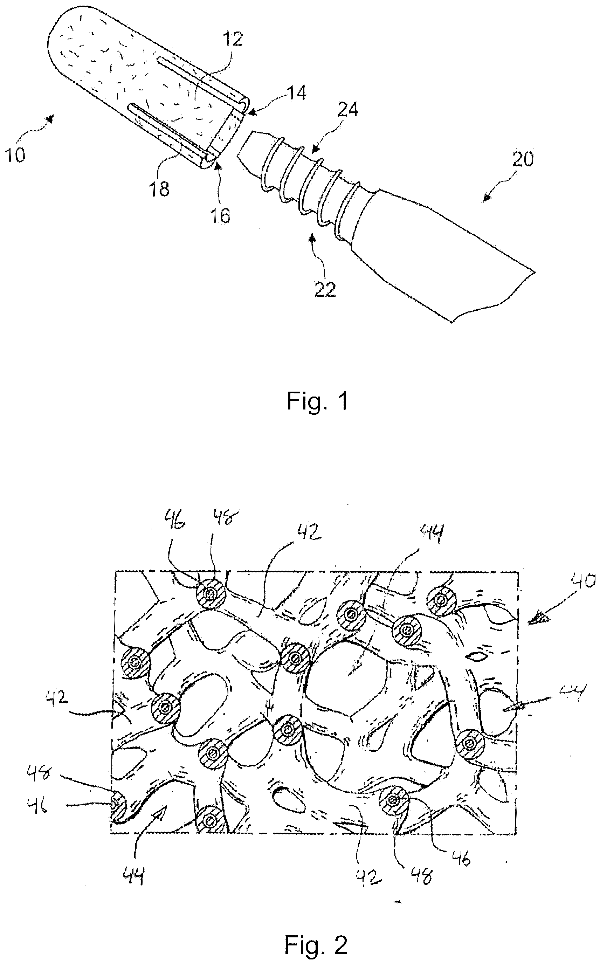

Fig. 1 zeigt ein Implantat, spezifischer zum Beispiel ein Schenkelhalsimplantat, umfassend eine Schenkelhalsschraube 20 und einen Dübel 10, der aus einem porösen Werkstoff wie zum Beispiel porösem Tantal gefertigt ist, das als Knochenersatzmaterial und/oder Zell- und Gewebeaufnahmefähiges biokompatibles Material geeignet ist. Der poröse biokompatible Werkstoff ist hierbei ähnlich zur Mikrostruktur der Spongiosa eines menschlichen Knochens ausgebildet und begünstigt das Verwachsen mit der Spongiosa, wenn er im Knochen verankert ist. Ein Beispiel eines solchen Werkstoffs ist das von Zimmer als Trabecular Metal™ vertriebene biokompatible Material. Dieses Material ist aus einem glasartigen Kohlenstoff-Schaumsubstrat mit Gitterstruktur gebildet, das mittels Gasphasenabscheidung (Chemical Vapour Deposition) mit einem biokompatiblen Material wie Tantal, Niobium oder einer Legierung mit Tantal und Niobium, beispielsweise einer Legierung aus Niobium, Hafnium und/oder Wolfram mit Tantal oder Hafnium und/oder Wolfram mit Niobium, beschichtet ist. In dieser Hinsicht wird Bezug genommen auf die US 5,282,861, die für den vorgeschlagenen Gegenstand geeignete biokompatible Materialen und deren Herstellung im Detail beschreibt, und deren Inhalt hiermit in diese Anmeldung aufgenommen wird. Wesentlich ist, dass das verwendete biokompatible Material geeignet ist, wenn es direkt im Knochengewebe, d.h. der Spongiosa platziert ist, als Prothese für Knochengewebe zu wirken und das Wachstum von Knochengewebe zu stimulieren. Hierbei wirken insbesondere die Poren des biokompatiblen Materials als Matrix zur Aufnahme und Verankerung von Knochengewebe, insbesondere Spongiosagewebe, und begünstigen damit ein besonders gutes Verwachsen eines aus einem solchen biokompatiblen Material hergestellten Implantats mit der Spongiosa.

Fig. 2 zeigt eine poröse Tantalumstruktur 40, wie sie sich besonders gut als biokompatibles poröses Material eignet. Die Struktur 40 weist eine große Anzahl von Stegen 42 auf, die offene Räume 44 dazwischen definieren. Jeder Steg 42 weist im Allgemeinen einen Kohlenstoffkern 46 auf, der durch einen dünnen Film aus Metall 48 wie beispielsweise Tantal bedeckt ist. Die offenen Räume 44 zwischen den Stegen 42 bilden eine Matrix von fortlaufenden Kanälen, die im Wesentlichen keine toten Enden besitzen, so dass das Wachstum von Knochengewebe durch die poröse Tantalstruktur nicht behindert wird. Die Struktur weist eine grosse Ähnlichkeit mit der Geometrie von Knochentrabekeln auf, und begünstigt so das Einwachsen von Knochengewebe weiter. Das poröse Tantal kann bis zu etwa 75% bis etwa 85% oder mehr leeren Raum darin aufweisen. Daher ist diese poröse

Tantalstruktur eine leichtgewichtige, aber dennoch starre, d.h. mit hohen Kräften belastbare poröse Struktur, die im Wesentlichen gleichförmig und konsistent in ihrer Zusammensetzung ist, und sehr der Struktur von natürlichem Knochengewebe ähnelt. Dadurch wird eine Matrix geschaffen, in die natürliches Knochengewebe einwachsen und ein daraus bestehendes Implantat im umgebenden Knochengewebe eines Patienten gut verankern kann. Die poröse Tantalstruktur 40 kann mit verschiedenen Dichten hergestellt werden, um optimal an das Knochengewebe angepasst zu werden, in welches das Implantat integriert wird; siehe hierzu auch die bereits erwähnte US 5,282,861.

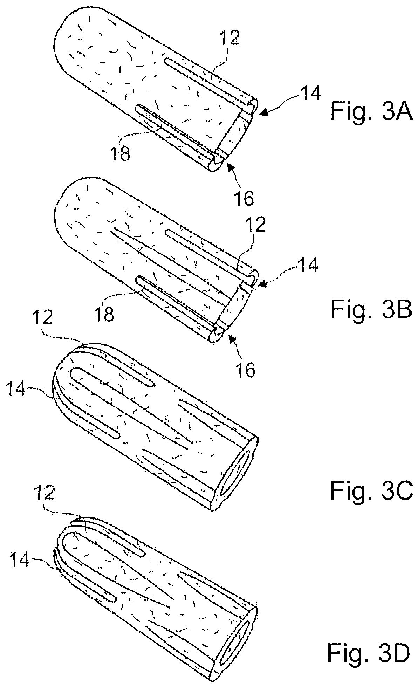

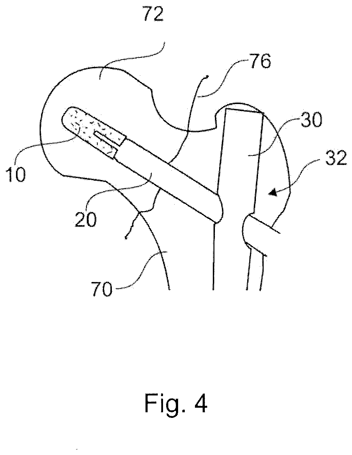

Der in Fig. 1 gezeigte Dübel 10 des Schenkelhalsimplantats weist vier Längsschlitze 12, 14, 16 und 18 auf, die dem Dübel 10 eine gewisse Elastizität verleihen, was das Einschrauben der Schenkelhalsschraube 20 erleichtert, und auch bis zu einem gewissen Maß ein Aufspreizen des Dübels 10 bei eingedrehter Schenkelhalsschraube 20 ermöglichen. Die Schenkelhalsschraube 20 weist an ihrem Ende 22 einen Gewindeabschnitt 24 auf, mit dem die Schraube 20 in den Dübel 10 eingeschraubt wird, sowie einen sich distal anschliessenden konischen bzw. kegelförmig ausgebildeten Bereich, der derart ausgebildet ist, dass es den Dübel 10 aufspreizen kann. Die Fig. 3A bis 3D zeigen verschiedene Ausgestaltungen des Dübels 10: bei den in den Fig. 3A und 3B gezeigten Ausgestaltungen sind Längsschlitze im distalen, oder, allgemeiner, im zum Grundkörper oder der Schraube hin orientierten Abschnitt des Dübels 10 vorgesehen, während die in den Fig. 3C und 3D gezeigten Ausgestaltungen im proximalen oder "spitzenseitigen" Abschnitt des Dübels 10 Längsschlitze zum Aufspreizen aufweisen. Operativ wird zunächst der Dübel 10 in eine entsprechende Bohrung im Femurkopf eingebracht, anschließend wird die Schenkelhalsschraube 20 in den im Femurkopf eingebrachten Dübel 10 eingedreht. Alternativ kann auch das Schenkelhalsimplantat umfassend den Dübel 10 und die Schraube 20 bereits vormontiert sein, d.h. die Schraube 20 bereits in den Dübel 10 soweit eingeschraubt sein, dass der Dübel 10 noch nicht aufgespreizt und daher leicht in die Bohrung im Femurkopf einführbar ist. Das implantat wird dann zum Beispiel eingeschlagen. Fig. 4 zeigt eine mit einem vorstehend beschriebenen Schenkelhalsimplantat versorgte proximale Femurfraktur 76. Im Femur 70 ist ein intramedullärer Marknagel 30 eingebracht. Im proximalen Abschnitt 32 des Marknagels 30 ist eine Schrägbohrung vorgesehen, durch welche die Schenkelhalsschraube 20 geführt und in der sie gelagert ist. Die Schenkelhalsschraube 20 ist in den im Femurkopf 72 eingebrachten Dübel 10 eingeschraubt, wodurch der Dübel 10 im Femurkopf etwas aufgespreizt wird.

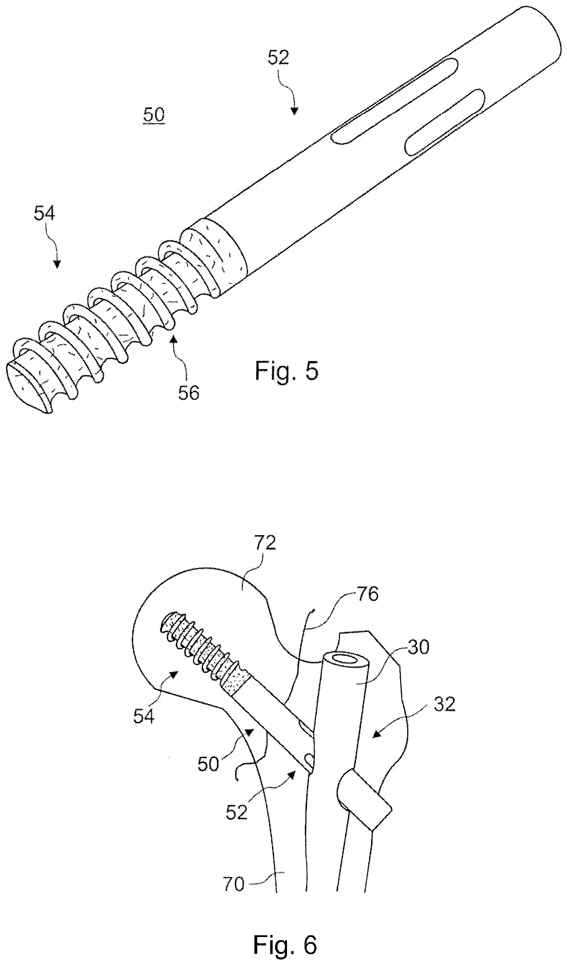

Fig. 5 zeigt als ein weiteres Ausführungsbeispiel eine hybride Schenkelhalsschraube 50 mit einem distalen Abschnitt 52, der zur Lagerung der Schraube 50 in einer Schrägbohrung eines intramedullären Marknagels dient, und mit einem proximalen Abschnitt 54, der aus einem porösen Werkstoff gefertigt ist und ein Spongiosagewinde 56 aufweist zum Einschrauben in die Spongiosa des Femurkopfs. Aufgrund der Porösität des proximalen Abschnitts 54 wird das Einwachsen von Knochengewebe in den proximalen Abschnitt 54 begünstigt, was eine besonders gute Verankerung der Schenkelhalsschraube 50 im Femurkopf bewirkt. Fig. 6 zeigt die Verankerung und Lage der Schenkelhalsschraube 50 im Femur 70 und Femurkopf 72.

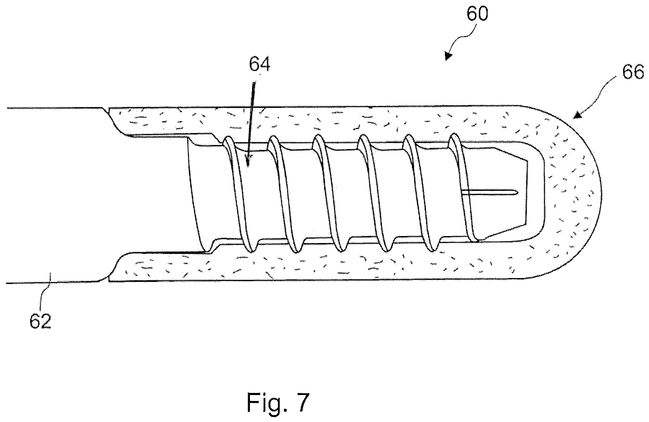

Fig. 7 zeigt beispielhaft in einem Schnitt eine mögliche Verbindung eines Grundkörpers 62 mit einem als Hülse oder Dübel ausgestalteten Verankerungsabschnitt aus porösem Material mittels eines Aussengewindes 64 auf einem Ende des Grundkörpers, also zum Beispiel auf dem proximalen Ende einer Schenkelhalsschraube. Der Verankerungsabschnitt weist dabei ein abgerundetes und geschlossenes Ende 66 auf.

Es ist festzuhalten, dass je nach Ausführungsform und Implantationstechnik ausschliesslich der Grundkörper oder aber auch das gesamte Implantat mitsamt dem Verankerungsabschnitt kannuliert sein kann, um eine geführte Implantation über einen Führungsdraht zu ermöglichen.

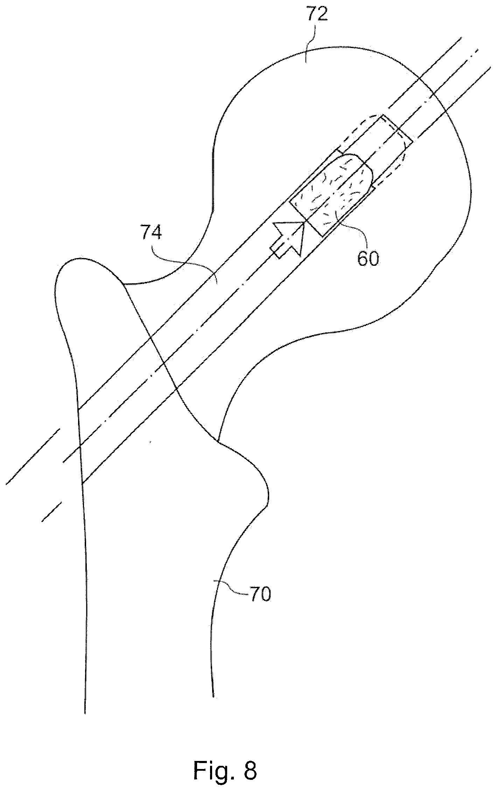

Fig. 8 zeigt eine Möglichkeit, wie die in Fig. 6 gezeigte Hülse 60 in den Femurkopf eingebracht werden kann: Zunächst wird mit beispielsweise einer abgestuften Reibahle bzw. einem abgestuften Räumer eine Bohrung 74 in der Spongiosa des Femurkopfs 72 zur Aufnahme der Hülse 60 und der Schenkelhalsschraube 62 erzeugt. Dann wird die Hülse 60 in die Bohrung eingeschlagen, bis sie fest in der Bohrung in einer gewünschten Tiefe im Femurkopf 72 sitzt. Anschließend wird die Schenkelhalsschraube 62 in die Hülse 60 eingeschraubt. Alternativ kann auch die bereits in die Hülse 60 eingeschraubte Schraube 62, als die vormontierte Kombination aus Hülse 60 und Schraube 62, in den Femurkopf 72 eingeschlagen werden.

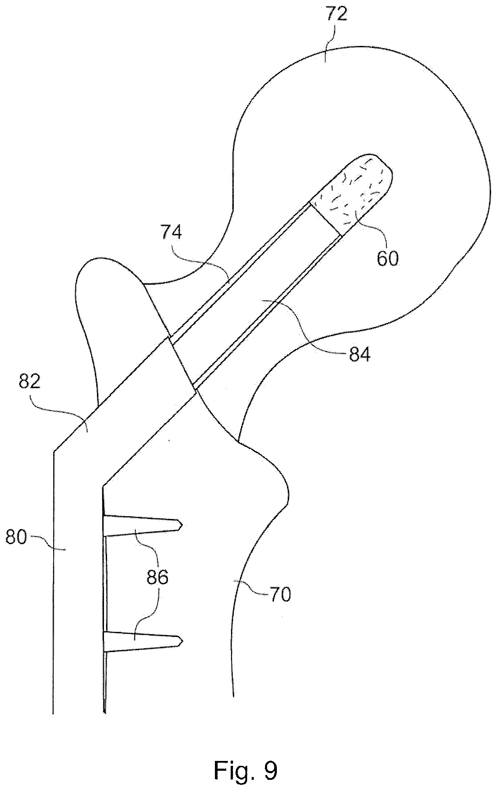

Fig. 9 zeigt die Versorgung einer Schenkelhalsfraktur des Femur 70 mit einem Implantat, das eine Platte 80 zur lateralen Fixierung am Femur 70 mittels Kortikalisschrauben 86 umfasst. Die Platte 80 weist eine Führungshülse 82 zur gleitenden Aufnahme eines Schenkelhalsimplantats auf. Die Führungshülse 82 ist in eine Bohrung im Femur 70 eingebracht. Das in der Führungshülse 82 gleitend gelagerte Schenkelhalsimplantat umfasst eine Schraube 84, die an ihrem proximalen mit einem Gewinde versehenen Ende in einer im Femurkopf 72 verankerten Hülse 60 aus porösem Tantal eingeschraubt ist. Dadurch ist die Schraube 84 am proximalen Ende fixiert, während sie an ihrem distalen Ende gleitend in der Führungshülse 82 gelagert ist. Die Schraube 84 weist an ihrem distalen Bereich, der sich in der Führungshülse 82 befindet, ein axiales Innengewinde auf, in das eine (nicht dargestellte) Kompressionsschraube im montierten Zustand des Implantats eingeschraubt ist. Die Kompressionsschraube dient vor allem dazu, die Gleitbewegung der Schraube 84 in der Führungshülse 82 zu limitieren und zumindest eine gewisse Kompressionswirkung auf die Fraktur auszuüben. Der Vorteil eines derartigen Implantats besteht vor allem darin, dass so gut wie keine Längskräfte durch die gleitend gelagerte Schraube 84 auf den Femur 70 und Femurkopf 72 übertragen werden und damit die auftretende Kompressionswirkung begrenzt werden kann. Eine Platte 80 mit Führungshülse 82 wird im Übrigen von der Anmelderin als Dynamische Hüftschraube DHS angeboten.

Im Folgenden wird beschrieben, wie die oben beschriebenen Schenkelhalsimplantate operativ implantiert werden können. Eine Implantierung kann offen, d.h. mittels Darstellung der proximalen Femurfraktur, oder minimal-invasiv unter Verwendung eines speziellen (nicht dargestellten) Zielgeräts in den Körper eines Patienten eingebracht werden. Zunächst wird ein intramedullärer Marknagel 30 in den Femur 70 eines Patienten eingebracht. Dies kann mit dem erwähnten Zielgerät erfolgen. Dann wird in die Spongiosa des Femurkopfs 72 eine Bohrung 74 zur Aufnahme des Schenkelhalsimplantats 10, 50 oder 60 eingebracht. Bei Verwendung der hybriden Schenkelhalsschraube 50 als Schenkelhalsimplantat wird die Schraube 50 dann durch eine Schrägbohrung im proximalen Abschnitt 32 des Marknagels 30 geführt und deren proximaler Abschnitt aus dem porösen biokompatiblen Werkstoff in die Bohrung 74 im Femurkopf 72 eingeschraubt. Bei Verwendung einer Kombination aus Dübel 10 bzw. Hülse 60 und Schenkelhalsschraube 20 bzw. 62 kann zunächst der Dübel 10 bzw. die Hülse 60 in die Bohrung 74 im Femurkopf 72 eingeschlagen werden, und anschließend die Schenkelhalsschraube 20 bzw. 62 durch eine Schrägbohrung im proximalen Abschnitt 32 des Marknagels 30 geführt und in den eingeschlagenen Dübel 10 bzw. die eingeschlagene Hülse 60 eingeschraubt werden. Alternativ kann auch die bereits vormontierte Kombination aus Dübel 10 bzw. Hülse 60 und Schenkelhalsschraube 20 bzw. 62 in die Bohrung 74 im Femurkopf 72 eingeschlagen werden und anschließend die Schenkelhalsschraube 20 bzw. 62 vollständig in den Dübel 10 bzw. die Hülse 60 eingeschraubt werden

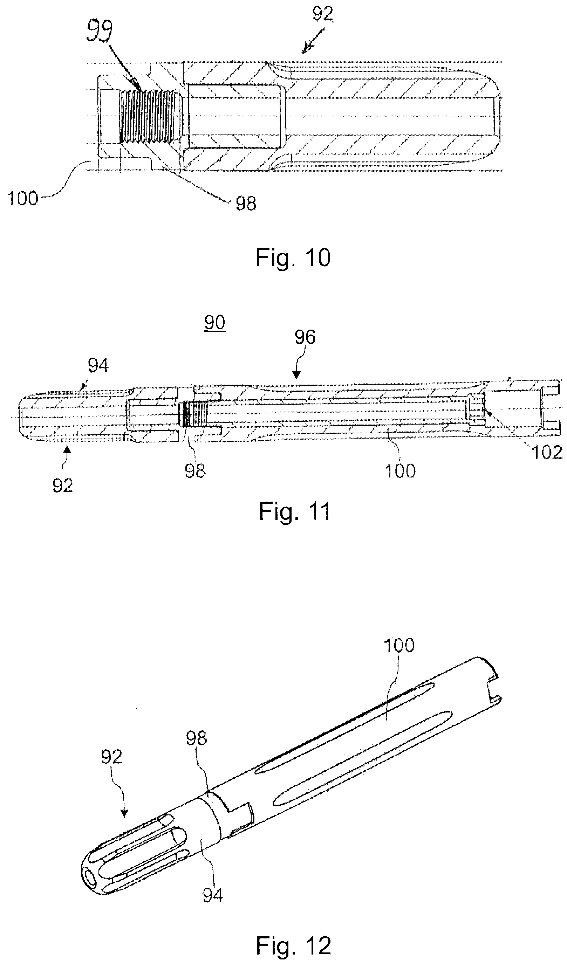

Fig. 12 zeigt ein weiteres Ausführungsbeispiel eines Implantats der angegebenen Art in Form eines Implantats, hier beispielsweise eines Schenkelhalsimplantats 90, das an seinem einen Ende im Femurkopf verankert und an seinem anderen Ende in einem intramedullaren Marknagel gelagert wird. Das dargestellte Implantat 90 umfasst einen Verankerungsabschnitt 92 und einen länglichen Grundkörper 96, der einen Schaft 100 umfasst. Der Schaft 100 ist beispielsweise aus Titanstahl zur Aufnahme in einer Schrägdurchbohrung des intramedullären Marknagels ausgebildet. Der Befestigungsabschnitt umfasst ein Substrat 98 und eine Ummantelung 94 aus porösem biokompatiblen Werkstoff, so, dass das Implantat in dem im Femurkopf verankerten Ende optimal in diesen verankert werden kann. Das Substrat weist ein Innengewinde 99 auf. Beide Teile 96 und 92 sind mittels einer Schraube 102, welche in das Innengewinde 99 eingeschraubt ist, miteinander verbunden. Das gesamt Implantat ist kannuliert. Zum Entfernen des Schaftes 100 wird die Schraube 102 gelöst und aus dem Implantat entfernt. Der Grundkörper 96 kann demnach aus dem Körper eines Patienten entfernt werden, wobei der Verankerungsabschnitt 92 des Implantats im Femurkopf verbleibt, da Knochengewebe mit dem porösen biokompatiblen Material verwachsen ist.

Figur 12 zeigt eine perspektivische Ansicht des Implantats aus den Figuren 10 und 11. Zu erkennen ist, dass der Verankerungsabschnitt mit Längsrillen versehen ist.

Die verwendeten Bezugszeichen bezeichnen die folgenden in der Zeichnung dargestellten Elemente :
- 10: Dübel aus porösem Tantal
- 12: Längsschlitz des Dübels
- 14: Längsschlitz des Dübels
- 16: Längsschlitz des Dübels
- 18: Längsschlitz des Dübels
- 20: Grundkörper, Schenkelhalsschraube
- 22: proximaler Endabschnitt der Schenkelhalsschraube
- 24: Gewindeabschnitt der Schenkelhalsschraube
- 30: intramedullärer Marknagel
- 32: proximaler Abschnitt des intramedullären Marknagels
- 40: poröse Tantalstruktur
- 42: Stege der porösen Tantalstruktur
- 44: offene Räume zwischen den Stegen
- 46: Kohlenstoffkern eines Stegs
- 48: Metallschicht
- 50: hybride Schenkelhalsschraube
- 52: distaler Abschnitt der hybriden Schenkelhalsschraube
- 54: proximaler Abschnitt aus porösem Tantal
- 56: Spongiosagewinde
- 60: Hülse oder Dübel
- 62: Schenkelhalsschraube
- 64: Gewinde der Schenkelhalsschraube
- 66: Endabschnitt der Hülse oder des Dübels
- 70: Femur
- 72: Femurkopf
- 74: Bohrung im Femurkopf
- 76: Proximale Femurfraktur
- 80: Platte zur lateralen Fixierung am Femur
- 82: Führungshülse für ein Schenkelhalsimplantat
- 84: Schenkelhalsschraube
- 86: Kortikalisschraube
- 90: mehrteiliges Schenkelhalsimplantat
- 92: Verankerungsabschnitt
- 94: Aussenseite aus porösem Werksteoff
- 96: Grundkörper
- 98: Substrat des Verankerungsabschnitts
- 99: Innengewinde
- 100: Schaft
- 102: Schraube

## Patentansprüche

1. Implantat zur Frakturversorgung, umfassend einen länglichen und insbesondere im Wesentlichen rotationssymmetrischen Grundkörper (20; 52; 62; 84; 96), der als Implantatschaft zur Gewährleistung der strukturellen Stabilität des Implantats ausgebildet ist, derart, dass Knochenfragmente während der Frakturheilung gegenseitig fixierbar sind, und einen Verankerungsabschnitt (10,54,60,92), wobei der Verankerungsabschnitt wenigstens teilweise eine Aussenfläche aus einem porösen Werkstoff (94) aufweist, und wobei der Verankerungsabschnitt in einem axialen Endbereich des Implantats angeordnet ist und lösbar mit dem Grundkörper verbindbar ist,
wobei der Grundkörper unmittelbar mit dem Verankerungsabschnitt durch Verschrauben lösbar verbunden ist oder der Verankerungsabschnitt ein Substrat (98) umfasst, an dessen Aussenseite der poröse Werkstoff (94) angeordnet und unlösbar mit dem Substrat verbunden ist und welches Mittel zur lösbaren Fixierung mit dem Grundkörper aufweist.

2. Implantat nach Anspruch 1, **dadurch gekennzeichnet, dass** es als Schenkelhalsimplantat, insbesondere zur Versorgung einer proximalen Femurfraktur oder einer Schenkelhalsfraktur, mit einem distalen und einem proximalen Ende ausgebildet ist, wobei der Verankerungsabschnitt das proximale Ende des Implantats bildet.

3. Implantat nach Anspruch 1 oder 2, **dadurch gekennzeichnet, dass** der poröse Werkstoff ein poröses Kohlenstoffsubstrat ist, das mit einem biokompatiblen Material beschichtet ist.

4. Implantat nach Anspruch 3, **dadurch gekennzeichnet, dass** das biokompatible Material Tantal, Niobium oder eine Legierung mit Tantal und Niobium, insbesondere eine Legierung aus Niobium, Hafnium und/oder Wolfram mit Tantal oder Hafnium und/oder Wolfram mit Niobium, ist, und das biokompatible Material insbesondere mittels einer Gasphasenabscheidung auf das poröse Kohlenstoffsubstrat aufgebracht ist.

5. Implantat nach einem der vorstehenden Ansprüche, **dadurch gekennzeichnet, dass** der Verankerungsabschnitt (10, 60) vollständig aus dem porösen Werkstoff besteht.

6. Implantat nach einem der vorstehenden Ansprüche, **dadurch gekennzeichnet, dass** das Substrat Mittel zur lösbaren Verbindung mit dem Grundkörper aufweist, wobei die Mittel insbesondere ein Innengewinde (99) und/oder ein Aussengewinde an einem axialen Ende des Verankerungsabschnitts sind.

7. Implantat nach einem der vorstehenden Ansprüche, **dadurch gekennzeichnet, dass** der Grundkörper als Schaft ausgebildet ist, der an einem Ende mit einem Gewinde zum Verbinden mit dem Verankerungsabschnitt ausgebildet ist.

8. Implantat nach Anspruch 7, **dadurch gekennzeichnet, dass** zwischen dem Schaft und dem Gewinde ein konischer, sich vom Gewinde zum Schaft hin im Durchmesser erweiternder, Bereich angeordnet ist.

9. Implantat nach einem der vorstehenden Ansprüche, **dadurch gekennzeichnet, dass** der Verankerungsabschnitt (54) an einem Aussenumfang ein Gewinde (56), insbesondere ein Spongiosagewinde, aufweist.

10. Implantat nach einem der vorstehenden Ansprüche, **dadurch gekennzeichnet, dass** der Grundkörper des Implantats zur Lagerung und/oder Führung des Implantats in einer Querbohrung eines intramedullären Marknagels (30) und/oder einer Führungshülse einer Frakturplatte ausgebildet ist.

## Claims

1. An implant for fracture treatment comprising an elongate and in particular substantially rotationally symmetrical base body (20; 52; 62; 84; 96) which is configured as an implant shaft to ensure the structural stability of the implant such that bone fragments can be fixed to one another during the fracture healing; and an anchorage section (10, 54, 60, 92), wherein the anchorage section has an outer surface of a porous material (94) at least in part, and wherein the anchorage section is arranged in an axial end region of the implant and is releasably connectable to the base body,
wherein the base body is directly releasably connected to the anchorage section by a screw connection or the anchorage section comprises a substrate (98) at whose outer side the porous material (94) is arranged and is non-releasably connected to the substrate and said substrate has means for the releasable fixing to the base body.

2. An implant in accordance with claim 1, **characterized in that** it is configured as a femoral neck implant, in particular for the treatment of a proximal femur fracture or of a femoral neck fracture, having a distal end and a proximal end, with the anchorage section forming the proximal end of the implant.

3. An implant in accordance with claim 1 or claim 2, **characterized in that** the porous material is a porous carbon substrate that is coated with a biocompatible material.

4. An implant in accordance with claim 3, **characterized in that** the biocompatible material is tantalum, niobium or an alloy with tantalum and niobium, in particular an alloy of niobium, hafnium and/or tungsten with tantalum or hafnium and/or tungsten with niobium; and the biocompatible material is in particular applied to the porous carbon substrate by means of gas phase deposition.

5. An implant in accordance with any one of the preceding claims, **characterized in that** the anchorage section (10, 60) consists completely of the porous material.

6. An implant in accordance with any one of the preceding claims, **characterized in that** the substrate has means for the releasable connection to the base body, with the means in particular being an internal thread (99) and/or an external thread at an axial end of the anchorage section.

7. An implant in accordance with any one of the preceding claims, **characterized in that** the base body is configured as a shaft which is formed at one end with a thread for connection to the anchorage section.

8. An implant in accordance with claim 7, **characterized in that** a conical region expanding in diameter from the thread toward the shaft is arranged between the shaft and the thread.

9. An implant in accordance with any one of the preceding claims, **characterized in that** the anchorage section (54) has a thread (56), in particular a spongiosa thread, at an outer periphery.

10. An implant in accordance with any one of the preceding claims, **characterized in that** the base body of the implant is configured for the journaling and/or guiding of the implant in a transverse bore of an intramedullary nail (30) and/or of a guide sleeve of a fracture plate.

## Revendications

1. Implant pour le traitement d'une fracture, comportant
un corps de base (20 ; 52 ; 62 ; 84 ; 96) allongé et en particulier sensiblement à symétrie de révolution, qui est réalisé sous forme de tige d'implant pour assurer la stabilité structurelle de l'implant, de telle sorte que des fragments osseux peuvent être fixés mutuellement pendant la guérison de la fracture, et
une portion d'ancrage (10, 54, 60, 92), la portion d'ancrage présentant au moins partiellement une surface extérieure en un matériau poreux (94), et la portion d'ancrage étant agencée dans une zone d'extrémité axiale de l'implant et pouvant être reliée de façon détachable au corps de base, dans lequel
le corps de base est relié directement à la portion d'ancrage de façon détachable par vissage ou la portion d'ancrage comprend un substrat (98) sur la face extérieure duquel est agencé le matériau poreux (94) et est relié de façon non détachable au substrat et qui comprend des moyens pour la fixation détachable avec le corps de base.

2. Implant selon la revendication 1,
**caractérisé en ce que**
il est réalisé à titre d'implant de col du fémur, en particulier pour le traitement d'une fracture proximale du fémur ou d'une fracture du col de fémur, comportant une extrémité distale et une extrémité proximale, la portion d'ancrage constituant l'extrémité proximale de l'implant.

3. Implant selon la revendication 1 ou 2,
**caractérisé en ce que**
le matériau poreux est un substrat poreux à base de carbone qui est revêtu d'une matière biocompatible.

4. Implant selon la revendication 3,
**caractérisé en ce que**
la matière biocompatible est du tantale, du niobium ou un alliage de tantale et de niobium, en particulier un alliage de niobium, de hafnium et/ou de tungstène avec du tantale, ou du hafnium et/ou du tungstène avec du niobium, et la matière biocompatible est déposée en particulier par dépôt en phase vapeur sur le substrat poreux à base de carbone.

5. Implant selon l'une des revendications précédentes,
**caractérisé en ce que**
la portion d'ancrage (10, 60) est constituée complètement en le matériau poreux.

6. Implant selon l'une des revendications précédentes,
**caractérisé en ce que**
le substrat comprend des moyens pour la liaison détachable avec le corps de base, les moyens étant en particulier un taraudage intérieur (99) et/ou un filetage extérieur à une extrémité axiale de la portion d'ancrage.

7. Implant selon l'une des revendications précédentes,
**caractérisé en ce que**
le corps de base est réalisé sous forme de tige qui est réalisée, à une extrémité, avec un filet pour la liaison avec la portion d'ancrage.

8. Implant selon la revendication 7,
**caractérisé en ce que**
entre la tige et le filet est agencée une zone dont le diamètre va en s'évasant depuis le filet vers la tige.

9. Implant selon l'une des revendications précédentes,
**caractérisé en ce que**
la portion d'ancrage (54) présente un filet (56), en particulier un filet spongieux, sur une périphérique extérieure.

10. Implant selon l'une des revendications précédentes,
**caractérisé en ce que**
le corps de base de l'implant est réalisé pour monter et/ou guider l'implant dans un perçage transversal d'un clou intramédullaire (30) et/ou d'une douille de guidage d'une plaque d'ostéosynthèse.
